# EUROPEAN PATENT APPLICATION

(11) **EP 4 599 751 A1**
(43) Date of publication of application: **13.08.2025**
(21) Application number: 25156791.3
(22) Date of filing: 10.02.2025
(51) Int. Cl.: A61B 1/00, A61B 1/06

(54) **SCOPE POSITION AND INTERFERENCE DETECTION**

(30) Priority: 12.02.2024 US 202463552449 P; 06.02.2025 US 202519047046
(71) Applicant: Arthrex, Inc, Naples, FL 34108-1945 (US)
(72) Inventor: Speier, Craig, Naples, 34108 (US); St. Clair, Gregory, Naples, 34108 (US); Jackson, Tim, Naples, 34108 (US); Holoien, Lee, Naples, 34108 (US); Olbrish, Ken, Naples, 34108 (US)
(74) Representative: Lohr, Jöstingmeier & Partner Patent- und Rechtsanwälte mbB

(57) **Abstract**

A method (100) for controlling a surgical scope (10) includes receiving an image feed (42) including a plurality of images from the surgical scope (10). One or more features (40) or characteristics of the plurality of images are then detected. In response to the one or more features (40) or characteristics, an operating state (36, 38) of the surgical scope (10) is identified in a deployed state (38) wherein the scope (10) is within a patient cavity (14) and alternatively in an undeployed state (36) wherein the scope (10) is outside the patient cavity (14).

## Description

### CROSS-REFERENCE TO RELATED APPLICATIONS

This application claims priority under 35 U.S.C. § 119(e) and the benefit of U.S. Provisional Application No. 63/5542,449 entitled SCOPE POSITION AND INTERFERENCE DETECTION, filed on February 12, 2024, by Speier et al., the entire disclosure of which is incorporated herein by reference.

### BACKGROUND

The disclosure generally relates to endoscopic surgical systems and, more particularly, to endoscopic systems and methods of operation that may detect the position of endoscopes and objects that may interfere with their operation. Developments in endoscopic devices have provided improved resolution, brightness, and resulting enhanced visibility allowing surgeons to improve surgical accuracy and the sophistication of procedures. An integral part of improving the visibility achieved in endoscopic systems may include increasing the intensity of light sources. The disclosure provides for methods and control systems that may control the operation of endoscopes to control the activation of light sources, which may allow surgeons and medical professionals to focus on managing the patient and procedure rather than manually managing the operation of the scope.

### SUMMARY

Managing the operation of light sources associated with endoscopes may increase tasks associated with surgical procedures and, in some cases, may result in conditions that could interrupt or interfere with the steps required to conduct a surgical procedure. For example, the continuing operation of a light source of a surgical scope may result in intense glare light and/or increased operating temperatures of the scope, particularly following the withdrawal of a scope from a patient cavity. Glare light may cause distractions, and elevated temperatures could cause discomfort or operation that is unsuitable in a surgical field. The disclosure provides for various systems and methods that may detect a position of the endoscope, which may be deployed or undeployed within a patient anatomy or cavity. In some implementations, the disclosure may further provide for the detection of one or more interference objects (e.g., drapes, bandages, gauze, tissue, etc.) in close proximity to a distal end of the surgical scope. In response to the detection of such objects when the surgical scope is undeployed or is located outside the patient anatomy, the disclosed methods may deactivate a light source of the scope.

In various implementations, the disclosure provides for a method for controlling a light source of a surgical scope in response to a plurality of images received via an image feed of the surgical scope. A control unit of the surgical scope may detect one or more features present in the plurality of images over time and determine whether the surgical scope is operating in a deployed state within a patient cavity or an undeployed state outside the patient cavity. In some cases, the operating method may monitor a lapsed time of the scope when identified in the undeployed state. In response to the lapsed time in the undeployed state exceeding a predetermined time, the operating method may deactivate the light source in the surgical scope. In other cases, the operating method may detect one or more interference objects within an interference proximity based on the one or more features identified in the plurality of images. In response to identifying the interference object at the interference proximity, the operating method may deactivate the light source of the surgical scope. These control techniques may be applied in combination to provide effective and intuitive control of the light source.

The deactivation of the light source responsive to the interference object in close proximity to the scope may bypass a countdown timer associated with the operation of the surgical scope to promptly deactivate the light source rather than awaiting the lapsed time of the countdown timer to exceed a predetermined time. In this way, the disclosure may provide for the deactivation of the light source in cases where the surgical scope is undeployed and further expedite the deactivation of the light source in cases where one or more objects or interference objects are identified at an interference proximity to the scope. As discussed herein, interference objects may correspond to various objects that may negatively interact with heat radiating from the light source of the surgical scope. Examples of interference objects may include a drape, paper or cloth products, bandages, gauze, wraps, biological tissue, or other objects ill-suited to exposure to elevated temperatures.

These and other features, objects and advantages of the present disclosure will become apparent upon reading the following description thereof together with reference to the accompanying drawings.

### BRIEF DESCRIPTION OF THE DRAWINGS

FIG. 1 demonstrates an environmental view of an endoscope deployed in a surgical procedure;
FIG. 2 is a process diagram demonstrating a scope feed output from a surgical scope;
FIG. 3A is a process diagram demonstrating a method for training a position detection model;
FIG. 3B is a process diagram demonstrating a method for training an interference detection model;
FIG. 4 is a flowchart demonstrating a light control routine; and
FIG. 5 is a block diagram demonstrating a surgical system in accordance with the disclosure.

### DETAILED DESCRIPTION

In the following description, reference is made to the accompanying drawings, which show specific implementations that may be practiced. Wherever possible, the same reference numbers will be used throughout the drawings to refer to the same or like parts. It is to be understood that other implementations may be utilized and structural and functional changes may be made without departing from the scope of this disclosure.

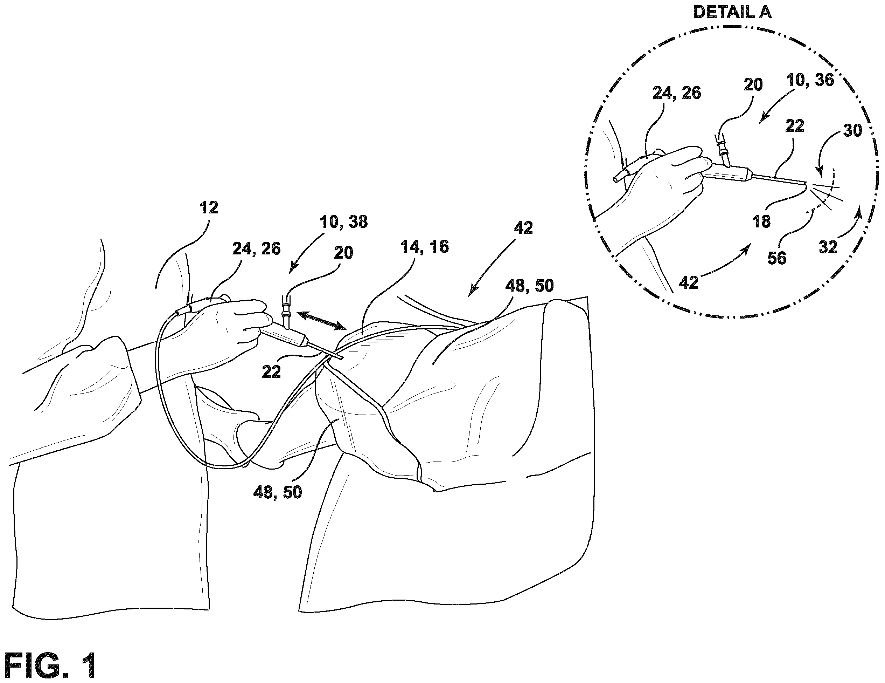

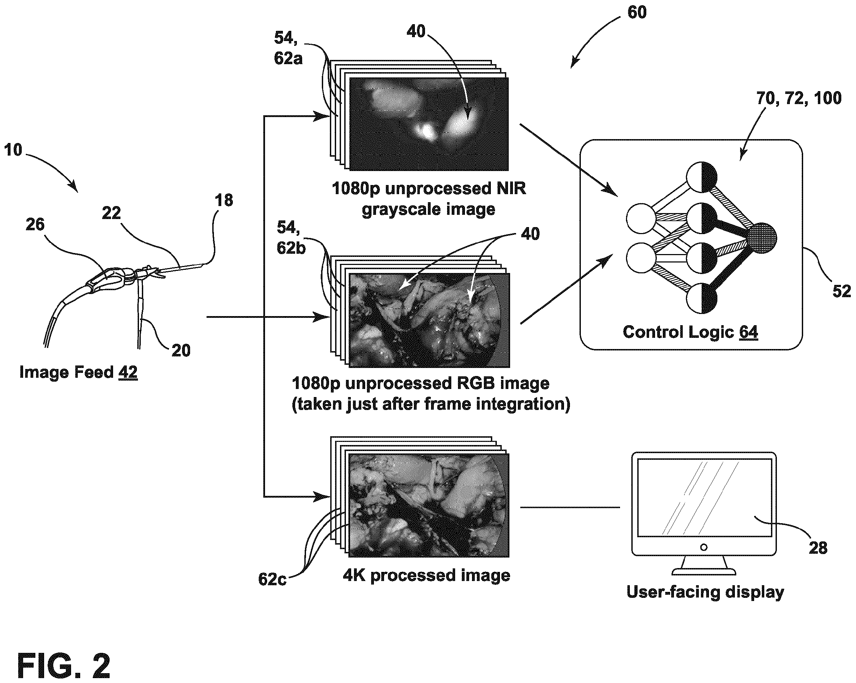

Referring generally to FIG. 1, an exemplary arthroscopic surgical procedure is shown demonstrating the deployment of a surgical scope 10 by a surgeon 12 in an opening or cavity 14 of a patient 16. In the example shown, the surgical scope or scope 10 may correspond to an arthroscope including a narrow distal end 18 deployed in the cavity 14 of the patient 16. In operation, light may be supplied to the distal end 18 from a light source 20 via a light guide through a scope body 22, which may be in the form of an elongated tubular structure. The light transmitted from the light source 20 may reflect from one or more features or anatomical structures present within the cavity 14 and reflect light back to the surgical scope 10. The reflected light is captured via an imager 24 of the scope 10, which may commonly be disposed within a camera body 26. As generally shown in FIG. 2 and discussed later in further detail, the imager 24 may be configured to capture an image feed comprising a plurality of images or frames illuminated by the light source 20 and depicting the one or more features in the patient cavity 14. The image feed may then be presented on a display 28 (FIG. 2), allowing the surgeon 12 to navigate one or more surgical tools in the cavity 14 to perform a surgical p roced u re.

Referring to FIGS. 1 and 2, at various times throughout the surgical procedure, the surgeon 12 may withdraw the distal end 18 of the surgical scope 10 from the patient cavity 14, such that a light emission 30 from the light source 20 maybe output from the distal end 18 of the scope 10 and into a local environment 32. As demonstrated in Detail A, the scope 10 is shown in an undeployed state 36 demonstrated in contrast to the deployed state 38 generally shown in FIG. 1. The result of the light emission 30 and operation of the light source 20 in the undeployed state 36 may result in highly distracting glare light as well as potential heat generation and elevated temperatures of the scope 10. The disclosure provides for an image-based control method that may infer an operating state of the scope 10 (e.g., undeployed 36, deployed 38) based on one or more features 40 presented in the image data or image feed 42. Responsive to the operating state, the control method may adjust or control the operation of the light source 20.

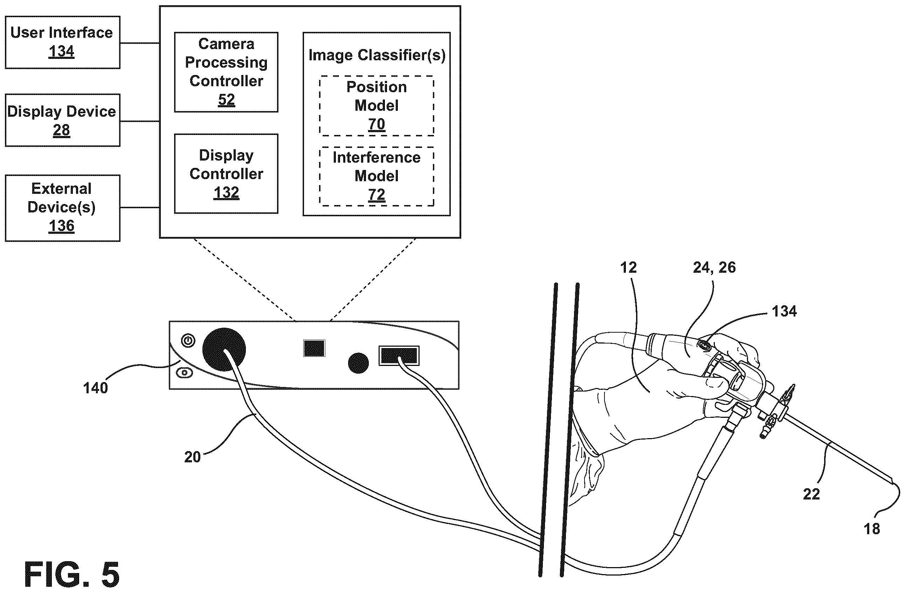

Still referring to FIGS. 1 and 2, the operation of the surgical scope 10 in a surgical field 46 may be accompanied by a variety of objects that may interfere with or otherwise respond negatively to the light emission 30 and/or excess heat generated by the scope 10. For example, as shown in FIG. 1, the surgical field 44 may include a variety of interference objects 48 that may preferably be avoided from contact and/or exposure to radiation and/or elevated temperatures associated with the operation of the light source 20. For example, interference objects 48 may include at least one of a drape 50, biological tissue, paper or cloth products, gauze, wraps, etc. In the example shown, a plurality of drapes 50 are positioned proximate to the cavity 14 of the patient 16 and may inadvertently come into contact with or in close proximity to the surgical scope 10 in the undeployed state 36. By determining the operating conditions or operating state of the scope 10, a controller 52 may control the light source 20 to avoid conditions that may interrupt or cause distractions during surgical procedures. The controller 52 is shown and described in further detail in FIG. 5 in reference to an exemplary camera system 130.

In operation, the interference objects 48 may be detected in the image feed 42 by a camera control unit or controller 52 to infer or deduce the operating state associated with the utilization of the scope 10. For example, the controller 52 may process images 54 or image frames of the image feed 42 with one or more trained models to determine whether the scope 10 is operating in the undeployed state 36 or the deployed state 38. Additionally, the controller 52 may apply a detection routine or trained model to infer or identify the interference objects 48 in the surgical field 44. The detection of the interference objects 48 may further determine whether the objects 48 are sufficiently close to the surgical scope 10 to be within an interference proximity 56. Based on these inferred operating conditions or states of the scope 10, the controller 52 may intuitively deactivate the light source 20 responsive to the deployment state 36, 38 and detection of the interference objects 48 within the interference proximity 56.

Referring now to FIG. 2, an image processing procedure 60 is depicted and described in reference to the image feed 42 output from a surgical scope 10. In the example shown, the image feed 42 may comprise image data representative of multiple light spectrums. In the example shown, the images 54 or image frames processed from the image feed 42 may include infrared (IR) image data 62a captured at a near IR spectrum, as well as visible light image data 62b captured in the visible light spectrum. The IR image data 62a and/or the visible light image data 62b may be supplied to the controller 52 and processed by control logic 64 that may include one or more trained computer vision models. The control logic 64 may include or more trained models that may infer or deduce the operating conditions or operating states of the scope responsive to the image feed 42. Additionally, the IR image data 62a and the visible light data 62b may be processed to generate formatted image data 62c for viewing on the display 28 for visualization, which may include portions or components of the IR image data 62a and the visible light data 62b.

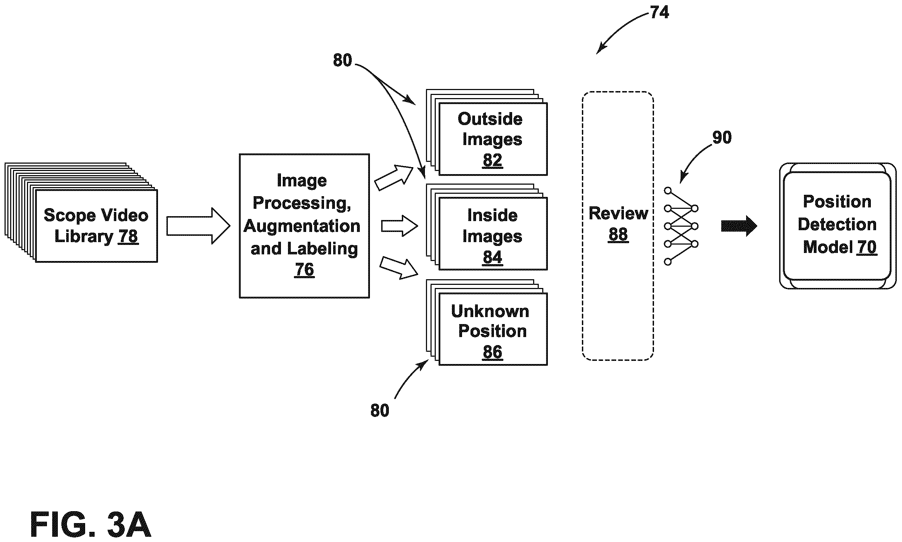

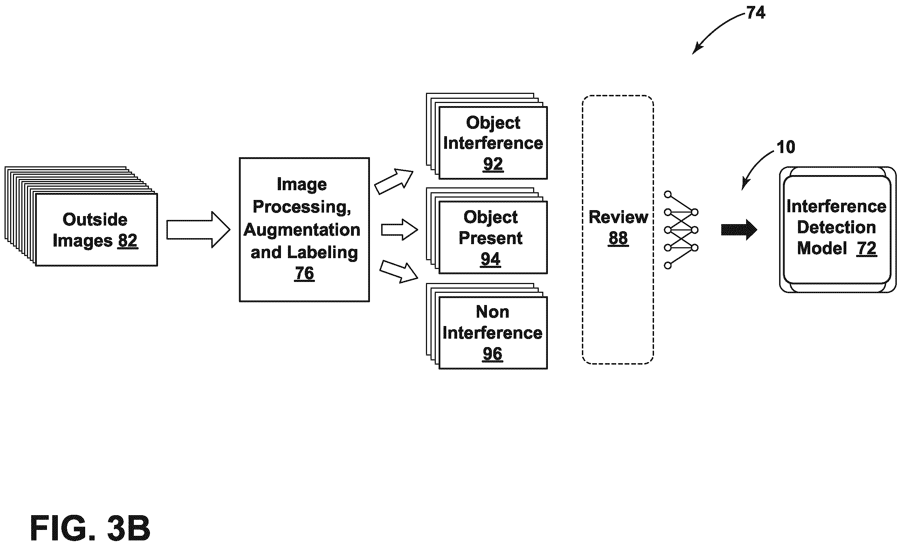

Referring now to FIGS. 3A and 3B, the computer vision models utilized to determine the operating states or conditions of the scope 10 are shown and discussed in further detail. For example, the detection models may include a position detection model 70 and an interference detection model 72. Each of the detection models 70, 72 may be applied by the control logic 64 of the controller 52 to determine the operating state of the surgical scope 10. As depicted in FIGS. 3A and 3B, a model training process is shown for each of the models 70, 72. Referring first to FIG. 3A, a model training routine 74 may generally include processing, augmenting, and/or labeling in step 76 of a plurality of images from a video library 78. In FIG. 3A, the data in the video library 78 may include representative images 54 captured of surgical scopes in the undeployed state 36 and the deployed state 38. The images 54 from the video library may generally be labeled or associated with the corresponding operating state 36, 38 by automated software or via direct human inspection. For example, in training the position detection model 70, the images of the video library 78 may be classified as outside images 82, inside images 84, and/or unknown images 86.

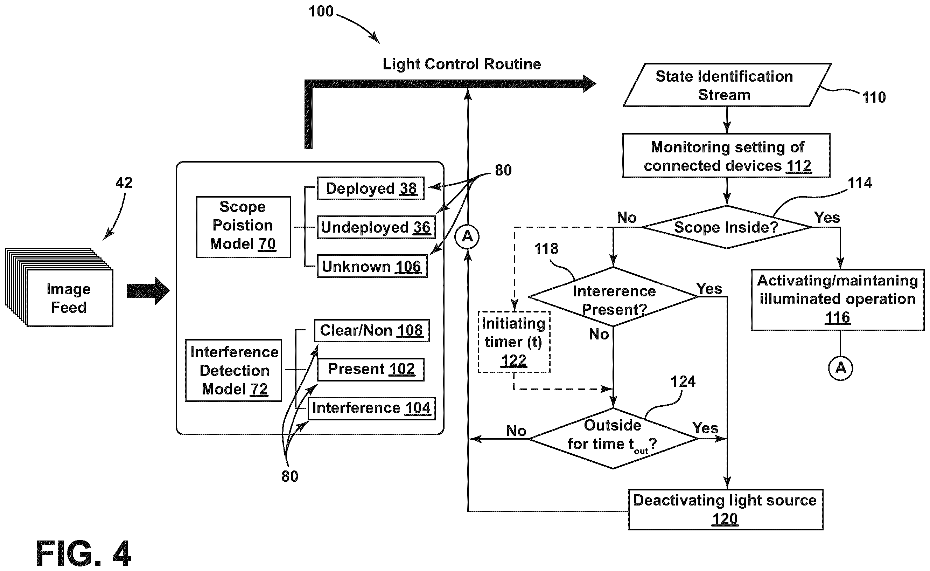

Following the labeling of the images 54 in step 76, the associated states of the images 82, 84, 86 may further be reviewed in step 88 to confirm that the conditions are representative of the corresponding operating states 80 (FIG. 4) of the surgical scope 10. Specifically, the outside images 82 may be confirmed to be representative of image data captured with the scope 10 in the undeployed state 36. The inside images 84 may be confirmed to correspond to image data representative of the scope 10 in the deployed state 38. Finally, the unknown images 86 may be confirmed to correspond to conditions that are not apparently representative of the undeployed or deployed states 36, 38, which may require a different control structure (e.g., manual control) of the light source 20. Once the representative images 82, 84, 86, are accurately labeled, they may be supplied to an untrained AI image processing model 90 to train and/or optimize the position detection model 70. The resulting position detection model 70 may be configured and trained to accurately distinguish whether the scope 10 is implemented in the undeployed state 36, deployed state 38, or other unknown states that may be tracked to disable one or more automated algorithms or controls or otherwise notify users of outlying image data.

Referring now to FIG. 3B, the model training for the interference detection model 72 may similarly be implemented to that previously discussed in reference to the position detection model 70. In operation, the training procedure for the interference detection model 72 may relate to the type of image data and associated conditions labeled in step 76. For example, the images labeled to train the interference detection model 72 may be limited to the outside images 82. In this way, the training procedure may focus the operation of the model 72 to distinguish characteristics of the outside images 82 representative of the operating conditions of the scope 10 in the local environment 32. As shown, the outside images 82 may be identified and labeled as object interference images 92, object present images 94, and noninterference images 96. Following the labeling procedure in step 76, the labels associated with the images 92, 94, 96 may be reviewed for accuracy in step 88. In this way, the images supplied to the untrained image processing model 90 may be confirmed to accurately exemplify the associate conditions shown in the images 92, 94, 96.

As with the position detection model 70, the images used to train the interference detection model 72 represent conditions associated with the operation of the scope 10. The noninterference images 96 may correspond to images that are free of objects identified in the local environment 32, while the object present images 94 may correspond to images where objects are present in the local environment 32 but not present within the interference proximity 56. Finally, the object interference images 92 may correspond to images where the associated features 40 indicate that one or more of the interference objects 48 (e.g., drapes 50, tissue, paper products, etc.) are present in the image data within the interference proximity 56. Once the images 92, 94, 96 are confirmed through the review step 88, the images 92, 94, 96 may be supplied to the untrained image processing model 90 to tune and train the interference detection model 72. Once effectively trained, the interference detection model 72 may distinguish interference from noninterference conditions of the scope 10 and be applied by the controller 52 to control the operation of the light source 20. In the exemplary control routine 100 later described in reference to FIG. 4, the position detection model 70 and the interference detection model 72 are applied in combination to intuitively control the operation of the light source 20 of the scope 10.

Referring again to FIGS. 3A and 3B, the image processing model 90 may correspond to a neural network that may be trained to recognize patterns in the images 54 representative of each of the states or operating conditions depicted in the labeled images 82, 84, 86, 92, 94, 96. By reviewing the respective labeled data relative the one or more features 40, conditions, levels of contrast, colors, line details, etc. present in the images 54, nodes of the untrained image processing model 90 may be arranged and attributed to a plurality of classifying functions and corresponding thresholds to determine patterns in the images 54 indicating or pointing to the corresponding operating states or conditions of the scope 10. Through one or more training operations, the training procedure may tune the image processing model 90 and verify a level of accuracy of the resulting trained models 70, 72 to deduce or infer the operating conditions of the scope 10. Such determinations may be processed rapidly by the controller 52 in part due to the nature of the detection being based on multiple layers of functions and weights attributed to the features 40 of characteristics of the images 54. In this way, the image data from the image feed 42 may be rapidly processed to indicate the state or condition of operation of the scope 10 and control the light source 20 to appropriately illuminate the field of view of the imager 24 or deactivate when warranted. Examples of untrained models for image classification may include, but are not limited to, the following: LeNet, AlexNet, ZF Net, GoogLeNet, VGGNet, ResNet, etc.

Though only discussed generally in reference to step 76, the image processing, augmentation, and enabling steps associated with the generation of the labeled image data may be beneficial to improve the robust operation of the resulting models 70, 72. For example, the images in the generic scope video library 78, as well as the outside images 82, may largely correspond to images processed and controlled to have a high level of clarity during surgical procedures. Accordingly, the images from the video library 78 and the outside images 82 may be supplemented with augmented images that may be adjusted to introduce variations of the existing image data that may assist the detection models 70, 72 in accurately identifying the corresponding operating states 80 with improved accuracy over a wide range of conditions. For example, the image data may be augmented to simulate and supplement the existing images with simulated zoom or blur situations, variations in color, variations in brightness and/or contrast, additive noise, or other variations that may be representative of scenarios that may be presented in the image feed 42. Additional examples of features or simulated image data may include images that are rigidly or elastically transformed as well as examples of images that demonstrate pixel dropout or occluded portions. Such features may be incorporated to supplement the captured images in the training libraries 78, 82 at random and at a defined probability to improve the detection capability and robust operation of the detection models 70, 72.

Referring now to FIG. 4, a flow chart is shown demonstrating a control routine 100 for the light source 20 of the surgical scope 10. As shown, the image feed 42 may be supplied to the position detection model 70 and the interference detection model 72. In operation, the controller 52 or camera control unit of the surgical scope 10 may process the image data concurrently or in rapid succession with each of the models 70, 72 to identify the corresponding operating states 80 of the surgical scope 10. As shown, the operating states 80 may include the undeployed state 36, the deployed state 38, the object present state 102, and the interference present state 104. Further, as previously discussed, the unknown deployment state 106 and the clear or no-object-present state 108 may also be identified by the models 70, 72 and applied to deactivate or adjust the operation of the control routine 100 or otherwise present messages alerting one or more users of the scope 10 of corresponding conditions.

As the images 54 of the image feed 42 are supplied to the models 70, 72, the states 36, 38, 102, 104, 106, 108 may be applied by the control routine 100 to intuitively operate the light source 20. In operation, the controller 52 may receive a state identification stream in step 110 indicating the states 80 or conditions determined by the models 70, 72. In addition to the operating states 80, the control routine 100 may further monitor various settings of the surgical scope 10 or other connected devices in step 112 as later described. In step 114, the routine 100 may query the state identification stream (110) to determine if the scope 10 is inside the patient cavity 14 in the deployed state 38. If the scope 10 is determined to be in the deployed state 38, the routine 100 may continue by activating or maintaining the illuminated operation of the light source 20 (116). Following step 116, the routine 100 or method may continue to Reference A and continue to monitor the state identification stream for updates. Alternatively in step 114, if the scope 10 is determined to be in the undeployed state 36, the routine 100 may continue to determine if an interference object 48 is present within the interference proximity 56, thereby activating the interference present state 104 (118). If the interference present state 104 is activated in step 118, the routine 100 may continue without a timed delay to step 120 to deactivate the light source 20. In this way, the routine may provide for the immediate or undelayed deactivation of the light source 20 in response to the presence of the interference object 48 detected within the interference proximity 56.

Concurrent to or in rapid succession with step 118, the routine 100 may initiate a timer (t) to monitor a lapsed time of the scope 10 identified in the undeployed state 36 (122). If the timer (t) exceeds a predetermined time threshold (tₒᵤₜ) in step 124, the routine may continue to step 120 to deactivate the light source 20. Accordingly, the controller 52 may respond differently in cases when the interference state 104 is identified. When the interference state 104 is not identified, the controller 52 may control the light source 20 on a timed delay to avoid situations where the light emission 30 results in glare light that may cause distractions in the surgical field 44.

As previously discussed in step 112, the routine 100 may further monitor various settings of the surgical scope 10 as well as one or more additional devices that may be in communication with the controller 52 to modify or control the operation of the light source 20. In one example, the controller 52 may monitor an exposure detected and associated with the operation of the imager 24 of the scope 10 to assist in the determination of the scope position in step 114. For example, a setting of the exposure (e.g., a minimum exposure time setting) over a predetermined time period may be characteristic of the scope 10 exposed to the local environment 32 in the undeployed state 36. Accordingly, in such cases, the exposure may be monitored by the routine to determine if it is characteristic of the deployed state 38 or the undeployed state 36. Such information may be utilized to assist in the determination of the scope position in step 114, particularly in cases where the scope position model 70 indicates that the scope position is unknown or in an unknown deployment state 106. Accordingly, the settings of one or more connected devices associated with the operation of the surgical scope 10 may be applied in combination with the states 80 identified by the detection models 70, 72 to improve the operation of the routine 100.

Referring now to FIG. 5, a block diagram of a camera system 130 is shown. As discussed throughout the disclosure, the system 130 may comprise a surgical scope 10 in communication with a camera controller 52. The surgical scope 10 may comprise the light source 20 at the image sensor or imager 24, a display controller 132, and a user interface 134. In various implementations, the scope 10 may correspond to an endoscope with an elongated scope comprising the narrow distal end 18 suited to various non-invasive surgical techniques. For example, the distal end 18 may include a diameter of less than 2 mm. As demonstrated, the scope 10 may be in communication with a control console 140 that may comprise the camera controller 52 and the display controller 132 via a communication interface. Though shown connected via a conductive connection, the communication interface may correspond to a wireless communication interface operating via one or more wireless communication protocols (e.g., Wi-Fi, 802.11 b/g/n, etc.).

The light source 20 may correspond various light emitters configured to generate light in the visible range and/or the near infrared range. In various implementations, the light source 20 may include light emitting diodes (LEDs), laser diodes, or other lighting technologies. In some cases, the light source 20 may correspond to a plurality of light sources configured to emit light in a plurality of wavelengths. For example, the light sources 20 may include a laser emitter configured to output emissions in the near infrared range including wavelengths from approximately 650 nm to 900 nm. Additionally, the light sources 20 may emit light in the visible spectrum including wavelengths ranging from approximately 380 nm to 700 nm. The imager or image sensor 24 may correspond to various sensors and configurations comprising, for example, charge-coupled devices (CCD) sensors, complementary metal-oxide semiconductor (CMOS) sensors, or similar sensor technologies.

The camera controller 52 may correspond to a control circuit configured to control the operation of image sensors 24 and the light source 20 by monitoring the operating states 80 of the scope 10 identified by the detection models 70, 72. The camera controller 52 may be in communication with the user interface 134, which may include one or more input devices, indicators, displays, etc. The user interface 134 may provide for the control of the scope 10 including the activation of one or more routines as discussed herein. The camera controller 52 may be implemented by various forms of controller, microcontrollers, application-specific integrated controllers (ASICs), and/or various control circuits or combinations.

Each of the display controller 132 and the camera controller 52, controller, or control unit may comprise various processors or memory devices and may be implemented as separate processing pipelines or via a combined processing architecture. The processor(s) may include one or more digital processing devices including, for example, a central processing unit (CPU) with one or more processing cores, a graphics processing unit (GPU), digital signal processors (DSPs), field programmable gate arrays (FPGAs), application specific integrated circuits (ASICs) and the like. In some configurations multiple processing devices are combined into a System on a Chip (SoC) configuration while in other configurations the processing devices may correspond to discrete components. In operation, the processor executes program instructions stored in the memory to perform the operations described herein. The memory may comprise one or more data storage devices including, for example, magnetic or solid state drives and random access memory (RAM) devices that store digital data. The memory may include one or more stored program instructions, object detection templates, image processing algorithms, etc. As shown, the position detection model 70 and the interference detection model 72 may be stored in the memory, such that the light control routine 100 may be processed by the camera system 130.

The display controller 132 may further comprise one of more formatting circuits, which may process the image data received from the scope 10, communicate with the processor, and output the enhanced image data to the display device 28. The formatting circuits 222 may include one or more signal processing circuits, analog-to-digital converters, digital-to-analog converters, etc. The display controller may also comprise a user interface 134, which may be separate or combined with the user interface of the camera controller 52. For example, the user interface(s) 134 may be in the form of an integrated interface (e.g., a touchscreen, input buttons, an electronic display, etc.) or may be implemented by one or more external devices 136 (e.g., a tablet) or peripheral devices (e.g., keyboard, mouse, etc.). In some implementations, the camera system 130 may also be in communication with an external server, which may correspond to a network, local or cloud-based server, device hub, central controller, or various devices that may be in communication with the camera controller 52 and/or the display controller 132 and, more generally, the camera system 10 via one or more wired (e.g., Ethernet) or wireless communication (e.g., Wi-Fi, 802.11 b/g/n, etc.) protocols. For example, the controllers 52, 132 may receive updates to the various trained models, operating modules, and routines as well as communicate sample image data from the scope 10 to a remote server for improved operation, diagnostics, and updates to the system.

According to some aspects of the disclosure, a method for controlling a surgical scope includes receiving an image feed comprising a plurality of images from the surgical scope; detecting one or more features or characteristics present in the plurality of images over time; and in response to the one or more features, identifying an operating state of the surgical scope in a deployed state wherein the scope is within a patient cavity and alternatively in an undeployed state wherein the scope is outside the patient cavity.

According to various aspects, the disclosure may implement one or more of the following features or configurations in various combinations:
- in response to identifying the scope in the undeployed state, monitoring a lapsed time of the undeployed state;
- in response to the lapsed time exceeding a predetermined time, deactivating a light source of the surgical scope;
- evaluating the one or more features to detect a presence of an interference object;
- in response to the operating state identified as the deployed state, suppressing a detection of the interference object identified responsive to the one or more features;
- the interference object comprises an extrinsic object that negatively interacts with heat radiating from a light source of the surgical scope;
- the interference object comprises at least one of a drape, biological tissue, paper or cloth products, gauze, wraps, etc.;
- wherein the interference object is further determined to be within an interference proximity, and in response to the one or more features identified as the interference object at the interference proximity, deactivating a light source of the surgical scope;
- wherein the deactivation of the light source in response to identifying the interference object at the interference proximity bypasses a countdown timer associated with the undeployed state deactivating the light source before a lapsed time of the countdown timer exceeds a predetermined time;
- wherein the interference object and the interference proximity are identified by a first image classification model trained from image data captured in the undeployed state;
- wherein the operating state is identified by a second image classification model trained from image data captured in both the deployed state and the undeployed state; and/or
- suppressing the identification of the interference object from the first image classification model in response to the operating state identified by the second image classification model.

According to another aspect of the disclosure, a control system is configured to control a light source configured to illuminate a field of view of a surgical scope. The control system comprises a camera control unit including a memory, wherein the camera control unit receives an image feed comprising a plurality of images and is in communication with the light source. The control unit is configured to detect one or more features or characteristics present in the plurality of images over time; evaluating the one or more features to detect an interference object within an interference proximity; and in response to the detection of the interference object, deactivate or suppress the activation of the light source.

According to various aspects, the disclosure may implement one or more of the following features or configurations in various combinations:
- the camera control unit is further configured to distinguish the interference object from other objects not presenting interaction hazards with an operation of the light source;
- wherein the camera control unit is further configured to, in response to the one or more features or characteristics, identify an operating state of the surgical scope in a deployed state wherein the scope is within a patient cavity and alternatively in an undeployed state wherein the scope is outside the patient cavity;
- wherein the camera control unit is further configured to, in response to identifying the scope in the undeployed state, monitoring a lapsed time of the undeployed state, and in response to the lapsed time exceeding a predetermined time, deactivating a light source of the surgical scope;
- wherein the camera control unit is further configured to, in response to the operating state identified as the deployed state, suppress the identification of the interference object identified responsive to the one or more features or characteristics;
- the interference object comprises an extrinsic object that negatively interacts with heat radiating from a light source of the surgical scope;
- the interference object comprises at least one of a drape, biological tissue, paper or cloth products, gauze, wraps, etc.; and/or
- the control system comprises the surgical scope and the surgical scope is an endoscope comprising an elongated insertion tube that is selectively deployed in the patient cavity.

According to yet another aspect of the disclosure, a control system configured to control a light source configured to illuminate a field of view of a surgical scope is disclosed. The control system comprises a camera control unit comprising a memory, wherein the camera control unit receives an image feed comprising a plurality of images and is in communication with the light source. The control unit is configured to detect one or more features or characteristics present in the plurality of images over time. In response to the one or more features or characteristics, the control unit identifies an operating state of the surgical scope in a deployed state wherein the scope is within a patient cavity and alternatively in an undeployed state wherein the scope is outside the patient cavity. The control unit may further evaluate the one or more features to detect an interference object within an interference proximity, and in response to the operating state identified as the deployed state, suppress the identification of the interference object identified responsive to the one or more features or characteristics. In response to the detection of the interference object in the undeployed state, the control unit suppresses the activation of the light source.

There is disclosed in the above description and the drawings a surgical camera system and method that fully and effectively overcomes the disadvantages associated with the prior art. However, it will be apparent that variations and modifications of the disclosed implementations may be made without departing from the principles described herein. The presentation of the implementations herein is offered by way of example only and not limitation, with a true scope and spirit being indicated by the following claims.

As used herein, words of approximation such as, without limitation, "approximately," "substantially," or "about" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as having the required characteristics or capabilities of the unmodified feature. In general, but subject to the preceding discussion, a numerical value herein that is modified by a word of approximation such as "approximately" may vary from the stated value by ±0.5%, ±1%, ±2%, ±3%, ±4%, ±5%, ±10%, ±12%, or ±15%.

Any element in a claim that does not explicitly state "means" for performing a specified function or "step" for performing a specified function, should not be interpreted as a "means" or "step" clause as specified in 35 U.S.C. § 112.

It will be understood that any described processes or steps within described processes may be combined with other disclosed processes or steps to form structures within the scope of the present device. The exemplary structures and processes disclosed herein are for illustrative purposes and are not to be construed as limiting.

It is also to be understood that variations and modifications can be made on the aforementioned structures and methods without departing from the concepts of the present device, and further it is to be understood that such concepts are intended to be covered by the following claims unless these claims by their language expressly state otherwise.

The above description is considered that of the illustrated embodiments only. Modifications of the device will occur to those skilled in the art and to those who make or use the device. Therefore, it is understood that the embodiments shown in the drawings and described above are merely for illustrative purposes and not intended to limit the scope of the device, which is defined by the following claims as interpreted according to the principles of patent law, including the Doctrine of Equivalents.

## Claims

1. A method (100) for controlling a surgical scope (10) comprising:
receiving an image feed (42) comprising a plurality of images from the surgical scope (10);
detecting one or more features (40) or characteristics present in the plurality of images over time;
in response to the one or more features (40), identifying an operating state (36, 38) of the surgical scope (10) in a deployed state (38) wherein the scope (10) is within a patient cavity (14) and alternatively in an undeployed state (36) wherein the scope (10) is outside the patient cavity (14);
evaluating the one or more features (40) to detect a presence of an interference object (48); and
in response to the operating state (36, 38) identified as the deployed state (38), suppressing a detection of the interference object (48) identified responsive to the one or more features (40).

2. The method (100) according to claim 1, further comprising:
in response to identifying the scope (10) in the undeployed state (36), monitoring a lapsed time of the undeployed state (36), and in response to the lapsed time exceeding a predetermined time, deactivating a light source (20) of the surgical scope (10).

3. The method (100) according to claim 1 or 2, wherein the interference object (48) comprises an extrinsic object that negatively interacts with heat radiating from a light source (20) of the surgical scope (10).

4. The method (100) according to any one of claims 1-3, wherein the interference object (48) comprises at least one of a drape, biological tissue, paper or cloth products, gauze, or wraps.

5. The method (100) according to any one of claims 1-4, further comprising:
wherein the interference object (48) is further determined to be within an interference proximity (56), and in response to the one or more features (40) identified as the interference object (48) at the interference proximity (56), deactivating a light source (20) of the surgical scope (10).

6. The method (100) according to claim 5, wherein the deactivation of the light source (20) in response to identifying the interference object (48) at the interference proximity (56) bypasses a countdown timer associated with the undeployed state (36) deactivating the light source (20) before a lapsed time of the countdown timer exceeds a predetermined time.

7. The method (100) according to claim 5, wherein the interference object (48) and the interference proximity (56) are identified by a first image classification model trained from image data (42) captured in the undeployed state (36).

8. The method (100) according to claim 7, wherein the operating state (36, 38) is identified by a second image classification model trained from image data (42) captured in both the deployed state (38) and the undeployed state (36).

9. The method (100) according to claim 8, further comprising:
suppressing the identification of the interference object (48) from the first image classification model in response to the operating state (36, 38) identified by the second image classification model.

10. A control system configured to control a light source (20) configured to illuminate a field of view of a surgical scope (10), the control system comprising:
a camera control unit (52) comprising a memory, wherein the camera control unit (52) receives an image feed (42) comprising a plurality of images and is in communication with the light source (20), wherein the control unit (52) is configured to:
detect one or more features (40) or characteristics present in the plurality of images over time;
in response to the one or more features (40) or characteristics, identify an operating state (36, 38) of the surgical scope (10) in a deployed state (38) wherein the scope (10) is within a patient cavity (14) and alternatively in an undeployed state (36) wherein the scope (10) is outside the patient cavity (14);
evaluate the one or more features (40) to detect an interference object (48) within an interference proximity (56);
in response to the operating state (36, 38) identified as the deployed state (38), suppress the identification of the interference object (48) identified responsive to the one or more features (40) or characteristics; and
in response to the detection of the interference object (48), deactivate or suppress the activation of the light source (20).

11. The control system according to claim 10, wherein the camera control unit (52) is further configured to:
distinguish the interference object (48) from other objects not presenting interaction hazards with an operation of the light source (20).

12. The control system according to claim 10, wherein the camera control unit (52) is further configured to:
in response to identifying the scope (10) in the undeployed state (36), monitoring a lapsed time of the undeployed state (36), and in response to the lapsed time exceeding a predetermined time, deactivating a light source (20) of the surgical scope (10).

13. The control system according to any one of claims 10-12, wherein the interference object (48) comprises an extrinsic object that negatively interacts with heat radiating from a light source (20) of the surgical scope (10).

14. The control system according to any one of claims 10-13, wherein the interference object (48) comprises at least one of a drape, biological tissue, paper or cloth products, gauze, wraps, etc.

15. The control system according to any one of claims 10-14, wherein the control system comprises the surgical scope (10) and the surgical scope (10) is an endoscope comprising an elongated insertion tube that is selectively deployed in the patient cavity (14).
